# EUROPEAN PATENT SPECIFICATION

(11) **EP 1 259 259 B1**
(45) Date of publication and mention of the grant of the patent: **08.06.2011**
(21) Application number: 01912024.5
(22) Date of filing: 02.02.2001
(51) Int. Cl.: A61K 39/39, A61K 39/00, A61K 39/145, A61K 31/573, A61P 37/04, A61P 37/08, A61P 11/08, A61P 19/02, A61P 31/16

(54) **MUCOSAL ADJUVANT FORMULATION**
ADJUVANSFORMULIERUNG ZUR SCHEIMHAUT-APPLIKATION
FORMULATION D'ADJUVANT MUCOSAL

(30) Priority: 23.02.2000 US 511582
(43) Date of publication of application: 27.11.2002
(73) Proprietor: Biotec Pharmacon ASA, 9008 Tromso (NO)
(72) Inventor: Raa, Jan, N-0851 Oslo (NO); Berstad, Aud K. H. Nat. Instituteof Public Health, N-0403 Oslo (NO); Bakke, Hilde Nat. Institute of Public Health, N-0403 Oslo (NO); Haneberg, Bjorn Nat. Institute of Public Health, N-0403 Oslo (NO); Haugen, Inger Lise Nat. Institute of Public Health, N-0403 Oslo (NO); HOLST, Johan National Institute of Public Health, N-0403 Oslo (NO); Janakova, Liba National Institute of Public Health, N-0403 Oslo (NO); Korsvold, Gro E. Nat. Institute of Public Health, N-0403 Oslo (NO); Oftung, Fredrik Nat. Institute of Public Health, N-0403 Oslo (NO)
(74) Representative: Gardner, Rebecca Katherine
(86) International application number: PCT/IB2001/000144
(87) International publication number: WO 2001/062283

(56) References cited:
- EP-A- 0 022 426
- EP-A- 0 045 718
- EP-A- 0 384 323
- WO-A1-95/30022
- DE-A- 3 019 614
- US-A- 5 032 401
- US-A- 5 401 727
- DATABASE BIOSIS [Online] BIOSCIENCES INFORMATION SERVICE, PHILADELPHIA, PA, US; 1976 HIRASE S ET AL: "STRUCTURAL STUDIES ON THE ANTI TUMOR ACTIVE POLY SACCHARIDES FROM CORIOLUS-VERSICOLOR BASIDIOMYCETES PART 2 STRUCTURES OF BETA-D GLUCAN MOIETIES OF FRACTIONATED POLY SACCHARIDES" Database accession no. PREV197662068171 XP002177061 & YAKUGAKU ZASSHI, vol. 96, no. 4, 1976, pages 419-424, ISSN: 0031-6903

## Description

### FIELD OF INVENTION

This invention relates generally to adjuvants for use with vaccines; more specifically adjuvants for use with mucosal vaccines which are non-immunogenic and serve to modulate immune reactions to antigens which are in contact with mucosal surfaces in humans.

### BACKGROUND OF INVENTION

Mucosal vaccines are rising in popularity on a global scale due in part to ease of administration of these vaccines as opposed to subcutaneous or other traditional means of administration the ease in facilitating self administration of mucosal vaccines as opposed to the traditional delays associated with assembling masses of people for traditional vaccine administration as well as the reduction and eventual elimination of hypodermic needles. An additional benefit of the development of effective mucosal vaccines is self administration, thus avoiding the necessity of trained personnel for traditional means of administration.

The mucosal surfaces are the most frequent entrance route of micro-organisms which cause disease in animals and humans. A specialized immune system, equipped with large amounts of lymphoid cells, protects these surfaces against invasion by micro-organism. This so-called mucosal immune system may generate strong specific immune responses to invading pathogenic micro-organisms, as well as to vaccine antigens which are administered onto the mucosal surfaces. There is a sound biological foundation for the growing interest both in human and veterinary medicine for mucosal vaccines which interact directly with this important part of the immune system. The rapid development in this field is also stimulated by the obvious advantages of vaccines that may be administered as nasal sprays or oral tonics, as compared to injection vaccines. However, mucosal vaccines will not become realistic alternatives to existing injection vaccines unless the efficacy of such vaccines can be improved. The use of helper substances, or adjuvants, is one of the most promising ways to enhance antibody response and clinical efficacy of mucosal vaccines.

Adjuvants have long been used with traditional vaccines to improve the immune response allowing for less vaccine to be used to produce the immune response.

Much of the work regarding the differences between the route of immunization comparing more traditional injection means with mucosal administration has been done and the conclusions widely accepted. Substantial evidence exists suggesting two immune systems; a peripheral immune system and a mucosal immune system. These systems are believed to operate separately and simultaneously in most species including humans.

The focus of the instant application is mucosal vaccines and more specifically a formulation of vaccine and adjuvant whereby the adjuvant should be non-toxic, should not disrupt the integrity of the mucosal membranes, should have physical-chemical characteristics which are suitable for transport through M-cells in mucosal tissues, activate the non-specific immunity without inducing antibody production against itself (the adjuvant), stimulate lymphocytes to produce antibodies specifically directed against antigens, not induce tolerance to antigens, act as a compatible carrier for antigens, enhance non-specific immunity and improve the clinical efficacy of specific vaccines.

The present invention discloses for the first time mucosal vaccine formulations with these desired characteristics and presents evidence of a true mucosal adjuvant effect without a simultaneous antibody response to itself. It is therefore envisaged that the mucosal adjuvant formulation presented herein may be a useful new contribution to human as well as veterinary medicine.

### DETAILED DESCRIPTION

The selective targeting of antigens and adjuvants to M-cells at the mucosal surfaces seems to be the most promising way to mount a secretory immune response, since these cells are specialized for sampling of antigens for direct exposure to immune cells in the mucosal tissues in the gastrointestinal and respiratory tract. Transport through M-cells is known to be more efficient for particulate than for soluble substances. In contrast to particles, soluble substances may adhere to and penetrate other parts of the epithelial lining as well, and therefore interfere with a broader range of immune competent cells, and as a consequence have a less predictable effect on the immune response than particulate antigens and particulate adjuvants. The transport efficacy of particles is affected by size and possibly also by their chemical nature. Particles in the size range of 1-10 micrometers seem to be most efficiently transported.

The search for safer and better vaccines, both for injection and for mucosal administration, has concentrated on defining and producing purified antigens that induce a high antibody response and high degree of protection. Most purified vaccine antigens are, however, weak immunogens when they are applied onto the mucosal surfaces. It has therefore become necessary to provide an adjuvant to enhance the ability of mucosal vaccine antigens to induce an efficient antibody response. There are a number of substances that have this ability. However, all adjuvants tested for their ability to enhance the efficacy of mucosal vaccines have one or more disadvantages. The most serious shortcomings of existing products are 1) unacceptable toxic effects, 2) induction of antibody production against the adjuvant itself, 3) induction of immunological tolerance to vaccine antigens.

Several substances with lipophilic or other characteristics which confer surfactant activity may increase absorption across mucosal membranes, thus increasing mucosal immunogenicity of vaccine antigens. Such excipients have been admixed to and tested as nasal adjuvants for diphtheria and tetanus toxoid, and compared with an aluminum-adsorbed vaccine given nasally in a human trial. A clear adjuvant effect was demonstrated, but local side effects were prominent, probably caused by the effect of the excipients on the epithelial membrane. The disruption of the membrane integrity also raises concerns regarding immune responses to other than the vaccine antigens concomitantly present at the mucosal surface.

Cholera is an exceptionally potent mucosal immunogen, as well as a potent adjuvant for mucosal and systemic antibody responses to unrelated antigens applied simultaneously and at the same site. The mechanism for its adjuvant activity is complex and may include enhanced antigen uptake across epithelial cells through its B-subunit, upregulation of antigen-presenting cells, and stimulation of B-cell switching. However, the direct effect of both CT and CTB (cholera toxin B) on T-cells *in vitro* is inhibitory, which has been suggested as a possible mechanism for adjuvanticity as well as induction of tolerance.

Coupling of antigen to recombinant CTB has been shown to enhance systemic tolerance to both CTB itself and the conjugated antigen after oral delivery. The apparent effect of CTB as an adjuvant on nasal delivery compared to the tolerogenicity on intestinal delivery may be due to trace amounts of the holotoxin contaminating commercial CTB preparations, restoring the adjuvant activity. However, a recent report comparing commercial and recombinant CTB showed adjuvant effects of recombinant CTB on nasal delivery, both when the vaccine antigen was coupled and admixed to CTB.

It has been shown that CT/CTB induces protective immunity after nasal delivery with several different vaccine antigens. Moreover, long-term immunological memory responses to CT as well as the associated antigen may be induced. Since the "wild" holotoxin is too toxic for use as a mucosal adjuvant in humans, new recombinants of the CT devoid of toxic properties have been developed. It remains unsettled, however, whether adjuvanticity of CT can be separated from its toxicity. CTB has been regarded as non-toxic, but induced substantial irritative effects when tried intranasally in high doses in humans.

CT induces immunity to itself as well as the admixed antigen, which may limit its adjuvant effect on repeated use. A few studies addressing this problem showed that pre-existing immunity to this adjuvant may inhibit serum responses to the antigen, more than local mucosal responses.

Recent studies using CT as adjuvant for whole-cell bacterial vaccine preparations have demonstrated further limitations in its adjuvant action, especially on nasal delivery. This confirms that the adjuvant mechanism of CT probably is different from the mechanism of particulate adjuvants, and that it has different adjuvant activity in different routes of immunization.

*Escherichia coli heat-labile enterotoxin* HLT has an A-B structure similar to CT, and 80% homology in amino-acid sequence. The mechanism of HLT adjuvanticity has not been studied as extensively as with CT, and may be somewhat different. Still, it has been suggested that HLT may abrogate mucosal tolerance through its effect on intraepithelial lymphocytes in the same way as CT. HLT is somewhat less toxic than CT, and, as with CT, trace amounts of the holo-toxin applied with the B-subunit may restore its adjuvanticity without toxicity.

HLT is a potent mucosal adjuvant, capable of inducing widely distributed, protective immune responses after intranasal delivery, and seems to be as effective as CT in inducing protective immunity. It is an effective adjuvant for serum and mucosal antibodies to more than one antigen administered simultaneously HLT. Non-toxic mutants with preserved adjuvanticity have been constructed. However, it remains to be settled whether these mutants can induce protective immunity, and if adjuvanticity can be separated from toxicity. When recombinant LTB a subunit of HLT, supplemented with a trace amount of recombinant LT was tried intra-nasally as adjuvant for influenza in a human study, the adjuvant effect seemed rather modest, and local undesired side effects were prominent.

Recombinant LTB coupled to antigen applied nasally can induce peripheral tolerance in the same way as CTB orally.

Other bacterial toxins or derivatives such as pertussis toxin also resembles cholera toxin, and nontoxic mutants have been produced. The non-toxic B subunit induced protective immunity against influenza on nasal delivery in one study, and a non-toxic mutant was compared to CT in another. The mutant was as least as efficient as the pertussis toxin in terms of protective immunity, although less immunogenic than CT in terms of systemic antibody enhancement. The adjuvant activity of pertussis toxin is well-known and is attributed to its mitogenic effects.

Muramyl dipeptide and derivatives are components derived from the cell wall of *mycobacteriae,* these substances have been shown to induce non-specific enhancement of immune responses when given nasally ahead of challenge.

The same principle of action has been demonstrated with chitin derivatives and the cytokine GM-CSF. The cytokine IL-2 has been tried intra-nasally encapsulated in liposomes and induced protective immunity, whereas IL-4 was inefficient. IL-2 was also superior to LTB in inducing protective immunity when combined with the vaccine antigen, despite comparable antibody levels. IL-5 and IL-6 have been entrapped in microspheres and enhanced mucosal IgA responses when applied ocularly.

### Liposomes

Liposomes are artificial lipid vesicles consisting of lipid layers, where the antigen may be encapsulated inside the aqueous compartment of the liposome, or associated with the antigen on the surface via surface-coupling techniques. Liposomes can be prepared easily and inexpensively on a large scale and under conditions that are mild to entrapped antigens. They do not induce immune responses to themselves, and are used in humans for parenterally administered drugs.

Their mucosal adjuvanticity was first ascribed to their particulate nature, thus implying carrier activity. Carrier function implies some kind of physical association between carrier and antigen, at least simultaneous administration of antigen and carrier. However, it was shown in one study that empty liposomes administered up to 48 hours prior to intranasal immunization with a subunit antigen could induce enhanced immunity.

When tried intranasally with different antigens, results on their adjuvant effect are conflicting. Most studies have demonstrated enhancement of systemic and secretory humoral responses, with protective function. However, it has been pointed out that these studies employed anesthesia during deposition of vaccine nasally, and this procedure allows the vaccine to spread throughout the whole respiratory tree, including lungs. Since this involves other lympho-epithelial structures than nasal-associated tissue, it may result in immune responses different from those induced when antigen is deposited in the nasal cavity only. In fact, it was suggested that liposomes may exert their adjuvant activity through their effect on alveolar macrophages, which normally are poor antigen-presenting cells. In those studies where liposomes were used intra-nasally in non-anesthetized animals, adjuvant effects were not consistently demonstrated. One study with liposomes prepared from viral membranes, which perhaps could be classified more as proteosomes than liposomes, demonstrated adjuvant effect for protection against influenza in non-anesthetized animals. Thus, the mechanism of any adjuvant activity of liposômes remains to be settled. Moreover, since liposomes seem safe in use, mucosal adjuvanticity should be evaluated in human studies as well.

### ISCOMS (Immune-stimulating complexes)

ISCOM matrix are negatively charged cage-like structures, created by the interaction of the glycoside saponins with cholesterol and phospholipid, into which antigens like proteins can be incorporated. They are widely used in veterinary vaccines, and have been shown to induce both humoral immunity as well as cytotoxic (CTL) T-cell-mediated responses. There has been some concern about their toxicity, possibly related to the content of the built-in adjuvant saponin, which has surface activity.

During recent years ISCOMS have been tried as mucosal adjuvants. However, adjuvant activity was not clearly demonstrated when nasal immunization was performed in the non-anesthetized experimental animal.

### Biodegradable polymeric microspheres

Biodegradable polymeric microspheres usually refers to particles consisting of polymers of DL-lactide and glycolide (PLG) that biodegrade *in vivo* into lactic and glycoloic acids by hydrolysis. Vaccine antigens can be entrapped into the particle, the size of which and consequently rate of degradation and body distribution may be controlled. PLG particles are non-immunogenic and have proven safe for human use during parenteral drug delivery. However, there are concerns regarding the encapsulation procedure which involves exposure to organic solvents with the potential of denaturation of encapsulated antigen, as well as traces of these solvents possibly left inside the particles. Changes of antigen may also occur in the acidic milieu inside the particle upon hydrolytic degradation in vivo.

Most studies of mucosal immunization with PLG-entrapped vaccine antigens have used the oral or gastric route, whereas nasal delivery has been used in some. They enhanced immune responses with protective function, including memory responses and in two studies cell-mediated responses also were induced. CTL responses, however, were more consistently induced with ISCOMS than PLG particles in a study with oral delivery of antigen. Another study demonstrated increased secretory antibodies with cytokines entrapped in the particles, applied to the eye.

Other particles such as poly-alkylcyanoacrylate particles are claimed to be biocompatible particles which adsorb proteins and have demonstrated adjuvanticity upon oral immunization. Microspheres prepared from derivatised a-acids have been prepared and also demonstrated adjuvant activity after oral administration. None of these particles have demonstrated induction of protective immunity, or have been tried in humans.

Proteosomes are particles, consisting of 60 to 100 nm vesicles, were originally prepared from outer membrane proteins from *meningococci.* Other membrane protein preparations from different bacteria or viruses may have similar characteristics and activities, and may also be called proteosomes. Proteosomes have proven safe for human use on parenteral administration as vaccine antigen, they are easily prepared on a large scale, and can be linked to synthetic peptides or proteins which are weak immunogens on mucosal surfaces. In addition to their particulate nature, they may exert adjuvant activity through their effect on B-cells. Their lipophilic nature may also imply enhancement of absorption through biological membranes.

Proteosomes have been tried as mucosal adjuvants on nasal delivery with bacterial lipo-polysaccharides, staphylococcal entero-toxin B and viral proteins. Some of these studies also showed that these particles could increase protective, long-lasting immune responses. They were at least as good adjuvants as cholera toxin. A recent study using outer membrane vesicles from meningococci as nasal vaccine in humans, demonstrated immunogenicity as well as absence of side effects. Moreover, bactericidal antibodies which correlate to clinical protection was induced, but with greater bactericidal activity than expected from ELISA measurements of specific antibody titres.

Inactivated bacteria also have potential as particulate adjuvants for mucosal application. This has been demonstrated in a recent study with whole *meningococci* and *pertussis* bacteria, where systemic and secretory antibodies to inactivated influenza virus were greatly enhanced when delivered intra-nasally in non-anesthetized mice. So-called bacterial ghosts, achieved by phage mediated lysis, have been mentioned as potential vaccine carriers for mucosal delivery.

Bacterial toxins are potent mucosal adjuvants, however, too toxic to be used in their native form. Non-toxic derivatives and soluble surface-active agents may have mucosal adjuvant activity due to increased uptake of the vaccine antigen through the epithelial mucosal membrane. However, there are unsettled questions concerning the mechanism for the adjuvanticity *versus* tolerogenicity for these agents, as well as for side effects when used in human trials. The M-cell mediated uptake of particulate adjuvants seems a more reliable way of inducing an immune response after mucosal delivery of non-proliferating vaccine antigens. Inert particles like liposomes, ISCOMs and biodegradable microspheres have a potential for human use through their safety record. However, their adjuvant effect on nasal delivery remains to be verified, preferably in human studies. Bacteria-derived particles, like proteosomes and whole-cell bacteria which have been tried nasally in humans without prominent side effects, are powerful mucosal adjuvants in animals. At present, such particles, although immunogenic to themselves, seem to be the most promising mucosal adjuvants.

US-A-5401727 discloses a process for stimulating the immune system of aquatic animals of the class Osteichthyes and subphylum Crustacea comprising administering an effective amount of a yeast cell wall glucan composed of glucopyranose units linked by predeterminately β-1,3 glycosidic bonds, having at least one branch therefrom of glucopyranose units linked by β-1,6 glycosidic bonds.

EP-A-0384323 discloses a β-1,3 glucan having a β-1,3-linked main chain with β-1,6-linked single glucose side chain administered to a fish treated with a vaccine.

US-A-5032401 discloses the incorporation of a drug into a whole β-glucan particle and the administration of it to an individual.

It would be an advancement in the art if the recognized problems associated with adjuvants and the use thereof in mucosal immunization or vaccinations could be overcome.

Accordingly, the present invention provides a glucan comprising glucose monomers linked together in branched chains by β-1,3 linkages and β-1,6 linkages for use in modulating human immune responses to a viral or bacterial vaccine which is brought into contact with or administered onto a mucosal surface, wherein said glucan is also administered onto a mucosal surface and wherein the glucan and the vaccine are co-administered. Surprisingly, the above glucan adjuvant has been shown to enhance the efficacy of mucosal vaccines formulations including but not limited to influenza virus vaccines, allergy vaccines and vaccines for use for the treatment of arthritis.

Mucosal vaccines and the adjuvant of beta-1,3/beta-1,6 polysaccharide (glucan) can be administered orally, nasally, rectally, vaginally, through gastric administration or any other means by which the vaccine and adjuvant are allowed to come into contact with mucosal surfaces.

### Preparation of Adjuvants for Mucosal

### Vaccinations (Administration)

**Micro-particulate product.** This product was prepared according to the procedure described in US Patent No: 5,401,727 by repeated extractions in alkali and acid of dry *Saccharomyces cerevisiae.* The extraction process described removes cytoplasmic components inside the yeast cells as well as the mannose containing polysaccharides and proteoglycans which are on the cell surface. The product prepared according to that previously described procedure, consists of a beta-1,3 beta-1,6-glucan with a particle size of 2-5 micrometers. The chemical structure of this micro-particulate beta-1,3 beta-1,6-glucan is characterized by 83% beta-1,3 linked glucose, 6% beta-1,6 linked and 5% beta-1,3,6 linked glucose, and it is a beta-1,3-glucan chain with beta-1,3,6-linked glucose as the branch points. The micro-particulate product was prepared as a 3% (w/v) suspension (stock suspension) in sterile distilled water and preserved with 0.3% formaldehyde. The experimental micro-particulate adjuvant product were prepared from this stock suspension by centrifugation and re-suspension in sterile distilled water to remove formaldehyde, followed by dilution to appropriate concentrations for mucosal administration.

**Enzyme treated micro-particulate product.** In this product, the side chains of beta-1,6-linked glucose in the micro-particulate product had been selectively removed by enzyme treatment with an enzyme which specifically acts on beta-1,6-linkages in a poly-glucose chain, according to Norwegian Patent No. 300 692: The micro-particulate product (0.2 grams), prepared as in US Patent No: 5,401,727, was suspended in 40 ml 50 mM ammonium acetate buffer at pH 5.0 and mixed with 20 units of the beta-1,6-glucanase enzyme. The mixture was continuously stirred for 6 hours at 37 degrees Celsius and the action of the enzyme stopped by boiling for 5 minutes. The residual enzyme treated particles were washed repeatedly in sterile distilled water by centrifugation and re-suspension. The resulting product is a branched beta-1,3-glucan with beta-1,3-glucan side chains connected by beta-1,6-linkaged at the branching points, and with no beta-1,6-linked glucose in the side chains which extend from the branching points.

**Solubilized product.** This product has the same chemical composition as the particulate product produced as described above. The difference is the particles have been broken up into smaller molecular aggregates which dissolve in water. The product was made according to the procedure described in Norwegian Patent No. 300 692: Micro-particulate product (2 grams) was suspended in I litre formic acid (90%) and heated to 80 degrees Celsius under continuous stirring. The suspension was cooled to 35 degrees Celsius and free formic acid removed by evaporation under vacuum. The residue was suspended in 0.5 litre distilled water and boiled for 3 hours. After cooling the suspension was filtered through a micro-pore filter (0.44 micron pore size), and freeze dried. The freeze dried product was suspended in 0.1 litre distilled water and dialysed (cut-off 5000 Dalton) against distilled water for 24 hours, before freeze drying. The dried product, dissolved in appropriate aqueous solution was used in the experiments.

**Solubilized enzyme treated product**. This product was made by solubilization of the enzyme treated micro-particulate product (6.1.2.) according to the same procedure as described in above.

### Experimental vaccines

To investigate the adjuvant effect of the beta-1,3 beta-1,6-glucan preparations described above, experimental influenza vaccine formulations were compared with regard to their ability a) to induce specific antibody response and b) to prime T-cells to proliferate when they are later exposed to vaccine antigens *in vitro.* The Control vaccines contained either heat inactivated whole influenza virus (=INV) without any adjuvant added or purified antigens ("split vaccine" = split-INV) of the same virus without adjuvant. The Experimental vaccines were made from the same influenza virus vaccine preparations, but admixed with adjuvants described above.

The animals used in the experiment were female BALB/c mice, 6-8 weeks of age (at the start of experiment).

### Experimental design

Groups of 6-10 mice were immunized intra-nasally with one of the vaccine formulations four times at weekly intervals. The vaccines were administrated as drops, with 30 micro-litres dose volumes in the nasal cavity of anesthetized mice. Non-immunized mice served as controls. One week after the last vaccine dose, samples of saliva, serum and spleen cells were collected for analysis of specific antibody responses and antigen specific T-cell proliferation.

### Analysis of B- and T-cell responses

IgG and IgA type of antibodies formed as a specific response to the influenza vaccine (INV) used in the present studies, were analyzed as described by enzyme-linked immuno-absorbent assay (ELISA), in both serum and saliva. Antigen-specific proliferation of CD4+ T-cells in spleen cell cultures *in vitro* were analyzed by measuring the rate of incorporation of C¹⁴ labeled thymidine into nucleic acids.

### Results

It is previous knowledge that all of the beta-1,3 beta-1,6-glucan products referred to above, enhance the disease resistance of animals by stimulating the innate and non-specific defense mechanisms (cf. US Patent No: 5,401,727; Norwegian Patent No. 300 693). It is also previous knowledge that beta-1,3 beta-1,6-glucans do not induce antibody production against themselves. It was previously postulated that beta-1,3 beta-1,6-glucans may act as adjuvants which enhance the efficacy of vaccines when injected together with vaccine antigens into animals. However, it has not been shown previously that administration of a beta-1,3 beta-1,6-glucan onto a mucosal surface of an animal, affects the specific immune system inside the body in such a way that it responds more actively to antigens which are administrated onto mucosal surfaces. The following examples show that the beta-1,3 beta-1,6-glucan products induce enhanced ability to produce specific antibodies against vaccine antigens which are co-administered onto mucosal surfaces, and furthermore, the beta-1,3 beta-1,6-glucan products prime T-cells in the spleen to respond more actively to later exposure of the same vaccine antigens.

### Example 1

**Table 1: The amount of serum antibodies (figures show kilo Units of IgG/ml) formed against all influenza virus "split vaccine" (split-INV) after nasal immunization of mice with the vaccine alone or with the same vaccine admixed with the experimental adjuvant.**

| Treatment | 2 micro-gram INV | 2 micro-gram INV + 75 microgram adjuvant | 20 micro-gram INV | 20 micro-gram INV + 75 microgram adjuvant |
|---|---|---|---|---|
| Average | 46 | 81 | 94 | 190 |
| Median | 40 | 66 | 79 | 205 |

The data in Example I show that the novel adjuvant formulation, in this case the micro-particulate product, enhances the production of serum (IgG) antibodies against influenza vaccine antigens when administered together with a non-proliferating influenza virus vaccine into the nasal cavity of mice.

### Example 2

**Table 2: The amount of secretory (saliva) antibodies (figures show Units of IgA/ml) against an influenza virus "split vaccine" (=split-INV) after nasal immunization of mice with the vaccine alone or with the same vaccine admixed with experimental adjuvant.**

| Treatment | 2 micro-gram INV | 2 micro-gram INV + 75 micro-gram adjuvant | 20 micro-gram INV | 20 micro-gram INV + 75 micro-gram adjuvant |
|---|---|---|---|---|
| Average | 50 | 119 | 95 | 152 |
| Median | 47 | 108 | 68 | 156 |

The data in Example 2 show that the novel non-proliferating adjuvant formulation, in this case the micro-particulate product, enhances the production of secretory antibodies (IgA) against influenza vaccine antigens when administered together with a non-proliferating influenza virus vaccine into the nasal cavity of mice.

### Example 3

**Table 3: The amount of secretory (saliva) antibodies (Units of IgA/ml) against whole influenza virus vaccine (= whole INV) after nasal immunization of mice with the vaccine alone or with the same vaccine admixed with experimental adjuvant.**

| Treatment | 125 micro-gram INV | 125 micro-gram INV + 75 micro-gram adjuvant |
|---|---|---|
| Average | 863 | 1122 |
| Median | 815 | 1125 |

The data in Example 3 show that the novel non-proliferating adjuvant formulation enhances the production of secretory antibodies (IgA) also against antigens in a whole influenza virus vaccine which is co-administered into the nasal cavity of mice.

### Example 4

**Table 4: The effect of the novel mucosal adjuvant on the ability of the influenza virus antigen to "prime" T-cells in the spleen of mice to respond to later exposure to the same antigen. The influenza vaccine (=INV) was given as a nasal spray, alone or co-administrated with 25, 75 and 150 micro-grams of the novel adjuvant. Three weeks later the ability of spleen T-cells to respond to the same influenza vaccine was measured in vitro as rate of proliferation, expressed as the rate of incorporation (cpm) of radioactive thymidine into nucleic acids.**

| Micro-gram INV | Control | INV | INV+25 micro-gram adjuvant | INV+75 micro-gram adjuvant | INV+150 micro-gram adjuvant |
|---|---|---|---|---|---|
| 0.25 | 0 | 1100 | 15500 | 29500 | 36500 |
| 2.5 | 0 | 1400 | 5300 | -- | 26500 |
| 25 | 0 | 3900 | 10200 | -- | 6050 |

The data in Example 4 show that when the experimental micro-particulate adjuvant formulation was administered together with a non-proliferating influenza vaccine into the nasal cavity of mice, it enhanced the ability of T-cells in the spleen to proliferate when such cells later were exposed to the same influenza vaccine. This shows that the novel adjuvant formulation is a true mucosal adjuvant for antigen specific T-cell response to an antigen administered onto a mucosal surface.

### Example 5

**Table 5: Proliferation response of spleen T-cells in mice which 3 weeks earlier had been vaccinated with a nasal influenza vaccine without adjuvant (Control) and with 75 micro-grams of the novel adjuvant (Experimental vaccine). The T-cells were stimulated in vitro with either 0.8 micro-grams/ml of the influenza virus vaccine or 0.8 micro-grams of the adjuvant. The response was measured as the rate of incorporation of radioactive (cpm) thymidine into nucleic acids.**

| Stimulated by | Control vaccine(INV) | Experimental vaccine |
|---|---|---|
| INV | 2800 | 23500 |
| Experimental adjuvant | 260 | 250 |

The data in Example 5 show that the mucosal adjuvant preparation does not prime spleen T-cells to respond to later were exposed to the same adjuvant, showing that the novel mucosal adjuvant preparation does not act as an antigen for T-cells. By comparison, the T-cells were primed to respond to later exposure of the influenza virus antigen in the vaccine, and this priming was markedly enhanced by the novel non-replicating mucosal adjuvant formulation, demonstrating its potency as adjuvant in mucosal vaccine formulations.

### Example 6

**Table 6: The effect of repeated nasal administration of a whole influenza vaccine (25 micro-grams/ml) without adjuvant (Control vaccine) or admixed with the novel adjuvant (75 micro-grantslml) (Experimental vaccine) on the production of secretory (saliva) antibodies against the influenza virus in mice. The mice were vaccinated nasally 4 times with one week intervals at start of the experiment (weeks 0,1,2,3), thereafter at week 8,9,10 and 11, thereafter at week 16,17,18 and 19. Saliva was collected after 4,8,12,16 and 20 weeks and analyzed for the content of anti-INVIgA (figures show U/ml).**

| | | 4 weeks | 8 weeks | 12 weeks | 16 weeks | 20 weeks |
|---|---|---|---|---|---|---|
| Control vaccine | Average | 500 | 516 | 1669 | 873 | 1275 |
| | Median | 425 | 548 | 1694 | 734 | 1453 |
| Experimental vaccine | Average | 552 | 633 | 2092 | 1456 | 1809 |
| | Median | 517 | 509 | 2136 | 1494 | 1668 |

The data in Example 6 show that the novel micro-particulate adjuvant product does not induce tolerance to influenza virus antigens, and that the effect of the mucosal adjuvant with regard to secretory IgA production is maintained for at least 20 weeks of repeated administration of the antigen onto the mucosal surfaces in the nasal cavity.

### Example 7

**Table 7: The effect of repeated nasal administration of whole influenza vaccine (25 micro-grams/ml) without adjuvant (Control vaccine) and with the novel adjuvant (75 micro-grams/ml) (Experimental vaccine) on the production of serum antibodies against the influenza virus in mice. The mice were vaccinated intra-nasally 4 times with one week intervals at start of the experiment (weeks 0,1,2,3), thereafter at week 8,9,10 and 11, thereafter at week 16,17,18 and 19. Blood samples were collected after 4,8,12,16 and 20 weeks and analyzed for the content of anti-INVIgG (figures show kU/ml).**

| | | 4 weeks | 8 weeks | 12 weeks | 16 weeks | 20 weeks |
|---|---|---|---|---|---|---|
| Control vaccine | Average | 102 | 262 | 937 | 1193 | 1380 |
| | Median | 102 | 284 | 842 | 954 | 1411 |
| Experimental vaccine | Average | 94 | 311 | 1436 | 1502 | 1830 |
| | Median | 100 | 319 | 1403 | 1606 | 1841 |

The data in Example 7 show that the novel mucosal adjuvant, in this case the micro-particulate product, does not induce tolerance to influenza virus antigens, and that the mucosal adjuvant effect with regard to serum IgG production is maintained for at least 20 weeks of repeated administration of the antigen onto the mucosal surfaces in the nasal cavity.

The above examples show representative results from experiments which support the claims set forth below. Corresponding results, but with different figures, were obtained with all four adjuvant preparations described herein.

### Other examples

The above examples show that the beta-1,3 beta-1,6-glucan products, when in contact with a mucosal surface, induce effects on such body-internal functions as the production of specific antibodies against virus vaccine antigens and antigen-specific priming of spleen T-cells. Corresponding results have been obtained also with bacterial antigens in mucosal vaccines, notably antigens in *Neisseria meningitidis* and *Bordetella pertussis.*

Furthermore, it is reported here, for the first time, that the same products also cause a notable and visible reduction of inflammatory and allergic reactions when administered onto mucosal surfaces. This is an apparent paradoxial response to a product which enhances non-specific immunity, and was therefore a non-expected observation.

## Claims

1. A glucan comprising glucose monomers linked together in branched chains by β-1,3 linkages and β-1,6 linkages for use in modulating human immune responses to a viral or bacterial vaccine which is brought into contact with or administered onto a mucosal surface, wherein said glucan is also administered onto a mucosal surface and wherein the glucan and the vaccine are co-administered.

2. The glucan for use according to claim 1 wherein the glucan is solubilised.

3. The glucan for use according to claim 1 or claim 2 wherein the viral vaccine is an influenza virus vaccine.

4. The glucan for use according to any one of the preceding claims wherein the administration is nasal.

5. The glucan for use according to claim 4 wherein the administration is by nasal spray or nasal drops.

6. The glucan for use according to any one of claims 1 to 3 wherein the administration is oral, vaginal, rectal or gastric.

7. The glucan for use according to claim 1 wherein the glucan is micro-particulate.

8. The glucan for use according to any one of the previous claims wherein the glucan and the vaccine are in admixture.

9. An adjuvant composition comprising a glucan as defined in claim 1, together with an influenza virus vaccine, formulated for administration as nasal spray or nasal drops.

10. Use of the glucan as defined in any one of the preceding claims for the manufacture of a mucosally administered adjuvant composition further comprising a viral or bacterial vaccine for use in modulating human immune responses to said vaccine which composition is brought into contact with or administered onto a mucosal surface.

## Patentansprüche

1. Glucan, das Glucose-Monomere enthält, die miteinander in verzweigten Ketten durch β-1,3-Bindungen und β-1,6-Bindungen gebunden sind, für die Verwendung bei der Modulation menschlicher Immunreaktionen auf einen viralen oder bakteriellen Impfstoff, der mit einer Schleimhautoberfläche in Kontakt gebracht wird oder darauf verabreicht wird, wobei das Glucan ebenfalls auf eine Schleimhautoberfläche verabreicht wird und wobei das Glucan und der Impfstoff gemeinsam verabreicht werden.

2. Glucan für die Verwendung nach Anspruch 1, wobei das Glucan löslich gemacht ist.

3. Glucan für die Verwendung nach Anspruch 1 oder nach Anspruch 2, wobei der virale Impfstoff ein Grippevirus-Impfstoff ist.

4. Glucan für die Verwendung nach einem der vorhergehenden Ansprüche, wobei die Verabreichung nasal erfolgt.

5. Glucan für die Verwendung nach Anspruch 4, wobei die Verabreichung durch ein Nasenspray oder durch Nasentropfen erfolgt.

6. Glucan für die Verwendung nach einem der Ansprüche 1 bis 3, wobei die Verabreichung oral, vaginal, rektal oder gastrisch erfolgt.

7. Glucan für die Verwendung nach Anspruch 1, wobei das Glucan in Form von Mikropartikeln vorliegt.

8. Glucan für die Verwendung nach einem der vorhergehenden Ansprüche, wobei das Glucan und der Impfstoff in Beimischung vorliegen.

9. Ergänzungszusammensetzung, die ein Glucan nach Anspruch 1 enthält, zusammen mit einem Grippevirus-Impfstoff, die für die Verabreichung als Nasenspray oder Nasentropfen formuliert ist.

10. Verwendung des Glucans nach einem der vorhergehenden Ansprüche bei der Herstellung einer auf die Schleimhaut verabreichten Ergänzungszusammensetzung, die ferner einen viralen oder bakteriellen Impfstoff für die Verwendung bei der Modulation menschlicher Immunreaktionen auf den Impfstoff enthält, wobei die Zusammensetzung mit einer Schleimhautoberfläche in Kontakt gebracht wird oder darauf verabreicht wird.

## Revendications

1. Glucane comportant des monomères de glucose liés ensemble dans des chaînes ramifiées par des liaisons β-1,3 et des liaisons β-1,6 en vue d'une utilisation pour moduler des réponses immunes humaines à un vaccin viral ou bactérien lequel est amené en contact d'une surface mucosale ou administré sur celle-ci, dans lequel ledit glucane est également administré sur une surface mucosale et dans lequel le glucane et le vaccin sont co-administrés.

2. Glucane en vue d'une utilisation selon la revendication 1, dans lequel le glucane est solubilisé.

3. Glucane en vue d'une utilisation selon la revendication 1 ou la revendication 2, dans lequel le vaccin viral est un vaccin contre l'influenzavirus.

4. Glucane en vue d'une utilisation selon l'une quelconque des revendications précédentes, dans lequel l'administration est nasale.

5. Glucane en vue d'une utilisation selon la revendication 4, dans lequel l'administration est effectuée par pulvérisation nasale ou gouttes nasales.

6. Glucane en vue d'une utilisation selon l'une quelconque des revendications 1 à 3, dans lequel l'administration est orale, vaginale, rectale ou gastrique.

7. Glucane en vue d'une utilisation selon la revendication 1, dans lequel le glucane est micro-particulaire.

8. Glucane en vue d'une utilisation selon l'une quelconque des revendications précédentes, dans lequel le glucane et le vaccin sont en adjuvant.

9. Composition d'adjuvant comportant un glucane comme défini dans la revendication 1, conjointement avec un vaccin contre l'influenzavirus, formulée pour l'administration sous forme de pulvérisation nasale ou de gouttes nasales.

10. Utilisation du glucane comme défini dans l'une quelconque des revendications précédentes pour la fabrication d'une composition d'adjuvant administrée par voie muqueuse comportant en outre un vaccin viral ou bactérien à utiliser pour moduler des réponses immunes humaines audit vaccin, laquelle composition est amenée en contact avec une surface mucosale ou administrée sur celle-ci.
